# EUROPÄISCHE PATENTSCHRIFT

(11) **EP 0 899 271 B1**
(45) Veröffentlichungstag und Bekanntmachung des Hinweises auf die Patenterteilung: **17.04.2002**
(21) Anmeldenummer: 98109186.1
(22) Anmeldetag: 20.05.1998
(51) Int. Cl.: C07K 1/00, C07K 14/54, C07K 14/715, A61M 3/00, A61K 38/20, A61K 38/17

(54) **Verfahren zur Induktion von therapeutische wirksamen Proteinen**
Process for inducing therapeutically active proteins
Procédé d'induction de protéines thérapeutiquement actives

(30) Priorität: 16.08.1997 DE 19735537
(43) Veröffentlichungstag der Anmeldung: 03.03.1999
(73) Patentinhaber: Orthogen Gentechnologie GmbH, 40210 Düsseldorf (DE)
(72) Erfinder: Reinecke, Julio, Dr., 50739 Köln (DE); Meijer, Hans, Dr., 50823 Köln (DE); Wehling, Peter, Dr.PD, 40597 Düsseldorf (DE)
(74) Vertreter: Schrell, Andreas, Dr.

(56) Entgegenhaltungen:
- EP-A- 0 088 971
- W.P. AREND ET AL.: "IgG INDUCTION OF IL-1 RECEPTOR ANTAGONIST PRODUCTION BY HUMAN MONOCYTES." IMMUNOLOGICAL REVIEWS, Nr. 139, 1994, Seiten 71-78, XP002086659 COPENHAGEN, DK
- V. RUIZ DE SOUZA ET AL.: "SELECTIVE INDUCTION OF INTERLEUKIN-1 RECEPTOR ANTAGONIST AND INTERLEUKIN-8 IN HUMAN MONOCYTES BY NORMAL POLYSPECIFIC IgG (INTRAVENOUS IMMUNOGLOBULIN)" EUROPEAN JOURNAL OF IMMUNOLOGY, Bd. 25, Nr. 5, Mai 1995, Seiten 1267-1273, XP002086660 WEINHEIM, DE
- CHEMICAL ABSTRACTS, vol. 125, no. 25, 16. Dezember 1996 Columbus, Ohio, US; abstract no. 325879m, L.S. ANDERSEN ET AL.: "IgG FOR INTRAVENOUS USE, AUTOLOGOUS SERUM AND PLASMA INDUCE COMPARABLE INTERLEUKIN-1 RECEPTOR ANTAGONIST LIBERATION FROM HUMAN MONONUCLEAR CELLS: AN IN VITRO PHENOMENON DEPENDING UPON PLASTIC ADHERENCE." Seite 1127; XP002086661 & AUTOIMMUNITY, Bd. 22, Nr. 2, 1995, Seiten 127-133,

## Beschreibung

Die vorliegende Erfindung betrifft ein Verfahren zur Herstellung von therapeutisch wirksamen. Proteinen sowie die dabei eingesetzten Mittel, insbesondere Spritzen.

Therapeutisch wirksame Proteine wie Erythropoietin, Insulin oder Interferone, sind seit langem bekannt. Viele dieser Proteine sind bereits als Arzneimittel zugelassen und werden dementsprechend häufig eingesetzt. Aufgrund der hohen mit der Entwicklung und Zulassung dieser Medikamente verbundenen Kosten besteht jedoch ein Bedarf an einfachen und kostengünstigen Alternativen zur Bereitstellung von therapeutisch wirksamen Proteinen. Zudem sind nicht alle therapeutisch wirksamen Proteine als Arzneimittel zugelassen. Gleichwohl besteht jedoch häufig der Bedarf, auch diese Proteine dem Patienten zu applizieren. Von besonderer Bedeutung sind dabei aufgrund ihrer mutmaßlichen guten Körperverträglichkeit autologe, das heißt körpereigene, Proteine. Zu diesen Proteinen gehören der Interleukin-1 Rezeptor-Antagonist, Interleukin 4, Interleukin 10 und der Tumor-Nekrose-Faktor-Rezeptor Typ I oder Typ II.

Die Stimulation von Monocyten durch adhärentes Immunglobulin G zur Bildung des Interleukin-1-Rezeptor-Antagonisten wird von Arend und Leung in Immunological Reviews (1994) 139, 71-78 und Moore et al. in Am. J. Respir. Cell Mol. Biol. (1992) 6, 569-575, beschrieben. Andersen et al. in Autoimmunity (1995) 22, 127-133 erläutert, daß der therapeutische in vivo zu beobachtende Effekt von Immunglobulin G nicht auf eine verstärkte Bildung von Interleukin-1-Rezeptor-Antagonist zurückgeführt werden kann und daß die in vitro Bildung des Interleukin-1-Rezeptor-Antagonisten (IRAP, IL-1ra) durch Monocyten in Abhängigkeit von an Polypropylen adsorbierten Serums- und Plasma-Bestandteilen stattfindet. Verfahren zur Herstellung unmittelbar in einer Therapie einsetzbaren IL-lra werden in diesen Druckschriften nicht beschrieben.

Das der vorliegenden Erfindung zugrundeliegende technische Problem besteht also darin, Verfahren und Mittel zur Herstellung therapeutisch wirksamer Proteine bereitzustellen, die als kostengünstige und schnell durchzuführende Alternative zum Einsatz und zur Herstellung konventioneller Arzneimittelpräparate dienen.

Die Erfindung löst dieses Problem, indem ein Verfahren zur Herstellung mindestens eines therapeutisch wirksamen Proteins oder eines Proteingemisches in einer Spritze bereitgestellt wird, wobei innere Strukturen der Spritze mit Induktoren, insbesondere Immunglobulinen, beschichtet werden, die Spritze mit einer Körperflüssigkeit eines Patienten gefüllt, inkubiert und das therapeutisch wirksame Protein in der Körperflüssigkeit gebildet wird. Die Erfindung sieht also in einem ersten Verfahrensschritt vor, daß innere Strukturen der Spritze mit Induktoren, insbesondere Immunglobulinen, beschichtet und diese dort immobilisiert werden. Nach der Beschichtung wird die Spritze in einem zweiten Verfahrensschritt mit einer Körperflüssigkeit, insbesondere Blut, Lymphflüssigkeit, Speichel oder Urin, gefüllt und inkubiert. Vorzugsweise wird die Körperflüssigkeit dem Patienten direkt mit der Spritze entnommen. Die an der inneren Struktur der Spritze immobilisierten Induktoren, insbesondere Immunglobuline, induzieren in der Körperflüssigkeit spezifisch, das heißt je nach eingesetztem Induktor, insbesondere- Immunglobulin, und eingesetzter Körperflüssigkeit, die Bildung therapeutisch wirksamer Proteine, die demgemäß in der in der Spritze vorhandenen Körperflüssigkeit angereichert beziehungsweise gebildet werden. Die so angereicherte Körperflüssigkeit kann in der Spritze steril gelagert und bei Bedarf dem Patienten direkt ohne weitere Behandlung oder beispielsweise nach Zentrifugation und/oder Sterilfiltration wieder zugeführt werden.

Die Erfindung sieht auch vor, daß die Bildung mehrerer Proteine in einer Körperflüssigkeit simultan induziert wird, so daß eine Körperflüssigkeit gebildet wird, die eine erhöhte Konzentration mehrerer Proteine aufweist.

Im Zusammenhang mit der vorliegenden Erfindung wird unter einer inneren Struktur einer Spritze jeglicher Bereich oder jegliche Struktur der Spritze verstanden, der in ihrem Inneren liegt, mit der aufzunehmenden Körperflüssigkeit in Kontakt kommt und mit Induktoren, insbesondere Immunglobulinen, beschichtet werden kann. In besonders vorteilhafter Weise ist die innere Struktur einer Spritze deren innere Oberfläche gegebenenfalls eine Oberfläche mit einer Strukturierung zur Oberflächenvergrößerung. Die innere Struktur kann jedoch entweder alternativ oder zusätzlich durch Partikel, Kugeln, Gele, Glaswolle, Granulat oder ähnliches gebildet sein, um die innere Oberfläche der Spritze zu vergrößern und so eine größere Beschichtungsoberfläche. für die Induktoren, insbesondere Immunglobuline, zur Verfügung zu stellen.

In besonders vorteilhafter Ausgestaltung der Erfindung ist vorgesehen, daß die Spritze, insbesondere die innere Struktur der Spritze, aus Polystyrol, Polypropylen, Glas oder einem ähnlichen Stoff hergestellt ist, das heißt aus diesen Stoffen besteht oder diese Stoffe im wesentlichen enthält, solange dieser Stoff Induktor-bindende, insbesondere Immunglobulin-bindende, Eigenschaften aufweist, also eine Adhäsion der Induktoren, insbesondere Immunglobuline, ermöglicht. In einer bevorzugten Ausführungsform der Erfindung ist vorgesehen, die innere Struktur einer Spritze herzustellen, indem die an sich nicht Protein-bindende innere Struktur der Spritze mit einer Protein-bindenden Beschichtung versehen wird.

Die vorliegende Erfindung ist vorteilhaft insofern, als daß ein einfach durchzuführendes Verfahren bereitgestellt wird, mit dem autologe, durch Induktion, insbesondere Immunglobulin-Induktion, herstellbare therapeutisch wirksame Proteine hergestellt werden können und in der so hergestellten Form, das heißt zusammen mit den anderen Bestandteilen der in der Spritze befindlichen Flüssigkeit dem Patienten direkt, das heißt ohne weitere Manipulation wie zum Beispiel Umfüllen in andere Behälter, wieder appliziert werden können. Gegebenenfalls kann zur Abtrennung von festen Bestandteilen eine Zentrifugation und/oder Sterilfiltration vorgesehen werden. Der Einsatz kommerziell erhältlicher oftmals teuerer Arzneimittel wird daher überflüssig. Zudem ist der Einsatz von therapeutisch wirksamen autologen Proteinen möglich, die bisher arzneimittelrechtlich noch nicht zugelassen und daher nicht verfügbar sind. Schließlich erweist sich die Erfindung als vorteilhaft, als daß eine Kontamination, Verunreinigung, Infektion oder ähnliches des therapeutisch wirksamen Proteins vermieden wird, die auf einer außerhalb des Patienten stattfindenden Arzneimittelherstellung beruht.

In einer besonders bevorzugten Ausführungsform der vorliegenden Erfindung ist das therapeutisch wirksame Protein Interleukin 4, Interleukin 10 oder löslicher Tumor-Nekrose-Faktor-Rezeptor Typ I oder Typ II, besonders bevorzugt der Interleukin-1 Rezeptor-Antagonist (IRAP oder IL-1ra).

In einer weiteren besonders bevorzugten Ausführungsform der vorliegenden Erfindung ist das Immunglobulin, mit dem die innere Struktur der Spritze beschichtet wird, Immunglobulin G. Im Zusammenhang mit der vorliegenden Erfindung wird unter einem Immunglobulin G isoliertes Immunglobulin G aber auch Immunglobulin G-haltige Immunkomplexe, Immunglobulin G-haltige Präparationen wie Sera, Plasma oder das Immunglobulin G Fc-Fragment beziehungsweise dieses enthaltende Präparationen oder Komplexe verstanden.

Die vorliegende Erfindung sieht in einer besonders bevorzugten Ausführungsform also ein Verfahren zur Herstellung des Interleukin-1 Rezeptor-Antagonisten vor, wobei die innere Struktur einer Spritze mit einem Induktor, insbesondere einem Immunglobulin, besonders bevorzugt Immunglobulin G, beschichtet, die Spritze mit einer Körperflüssigkeit, vorzugsweise Blut, gefüllt, inkubiert und der Interleukin-1 Rezeptor-Antagonist in der Körperflüssigkeit gebildet und angereichert wird. Durch die Bindung oder Adhäsion des Induktors, insbesondere Immunglobulin G, an die Oberfläche der inneren Struktur der Spritze ist dieses in der Lage, die im Blut vorhandenen Monocyten zur Bildung des Interleukin-1 Rezeptor-Antagonisten zu anzuregen, so daß dieses im Blut angereichert wird. Das sich in der Spritze befindende Blut kann nach Inkubation, das heißt nach Anreicherung des Interleukin-1 Rezeptor-Antagonisten, direkt, ohne weitere Manipulation wie zum Beispiel Umfüllen, wieder dem Patienten zugeführt werden, dem das in die Spritze eingefüllte Blut entnommen worden war. Vorteilhafterweise kann zur Abtrennung fester Bestandteile, wie Zellen, eine Zentrifugation und/oder Sterilfiltration vorgesehen werden. Die Erfindung sieht also auch vor, daß dem Patienten das Blut mittels der Induktor-beschichteten, insbesondere Immunglobulin G-beschichteten, Spritze entnommen, das Blut in der Spritze inkubiert und nach IL-1ra-Bildung dem Patienten wieder mit der Spritze zugeführt werden kann. Eine derartige Vorgehensweise ist zum Beispiel besonders vorteilhaft im Bereich der Neuroorthopädie, das heißt beispielsweise bei neurologisch bedingten Rückenbeschwerden. Für die Behandlung derartiger Beschwerden kam bisher nur eine Bandscheibenoperation, Cortisonbehandlungen, Spülungen mit Kochsalzlösungen oder ähnliches in Betracht. Die vorliegende Erfindung erlaubt nun die einfache und kostengünstige Bereitstellung eines therapeutisch wirksamen Proteins zur Behandlung der Beschwerden.

Die Erfindung sieht in einer weiteren bevorzugten Ausführungsform vor, daß die innere Struktur der Spritze zusätzlich mit Anticoagulantien beschichtet wird, insbesondere Heparin. Erfindungsgemäß kann auch vorgesehen sein, die Anticoagulantien nicht als Beschichtung, sondern ungebunden im Behälter einzusetzen, zum Beispiel in lyophilisiertem oder flüssigem Zustand in die Spritze zu geben.

Die Erfindung sieht in einer weiteren bevorzugten Ausführungsform vor, die Inkubation der Körperflüssigkeit in der Spritze über einen Zeitraum von 12 bis 72 Stunden, vorzugsweise bei Raumtemperatur bis 41°C, insbesondere bei 37°C, durchzuführen.

Die Erfindung sieht in einer Ausgestaltung der Erfindung auch vor, daß nach Bildung des therapeutisch wirksamen Proteins in der Körperflüssigkeit die Körperflüssigkeit weiter behandelt wird, um beispielsweise bestimmte Bestandteile dieser abzutrennen, zum Beispiel Blutplasma oder Blutplättchen. Diese Abtrennung kann in bevorzugter Ausführungsform der Erfindung durch Zentrifugation durchgeführt werden.

Die Erfindung betrifft in einer weiteren Ausführungsform ein Verfahren zur Herstellung einer zur In-vitro-Induktion von therapeutisch wirksamen Proteinen, insbesondere des Interleukin-1 Rezeptor-Antagonisten, geeigneten Spritze, wobei ein Induktor, vorzugsweise ein Immunglobulin, insbesondere Immunglobulin G, in die Spritze mit proteinbindender innerer Struktur eingebracht und so inkubiert wird, daß der Induktor, insbesondere das Immunglobulin G, an die innere Struktur bindet.

Die Erfindung betrifft selbstverständlich auch die so hergestellte Spritze, die in besonders bevorzugter Ausführungsform aus Polystyrol, Polypropylen oder Glas hergestellt ist, wobei sich die Spritze durch eine Beschichtung ihrer inneren Struktur mit einem Induktor, insbesondere einem Immunglobulin, vorzugsweise mit Immunglubolin G, auszeichnet.

Die Erfindung betrifft auch die Verwendung von Immunglobulinen, insbesondere Immunglobulin G, zum Beschichten von inneren Strukturen von Spritzen, vorzugsweise aus Polystyrol, Polypropylen oder Glas, zur In-vitro-Induktion von therapeutisch wirksamen Proteinen, vorzugsweise dem Interleukin-1 Rezeptor-Antagonisten.

Weitere vorteilhafte Ausgestaltungen der Erfindung ergeben sich aus den Unteransprüchen.

Die Erfindung wird anhand von Figuren und Ausführungsbeispielen näher erläutert.

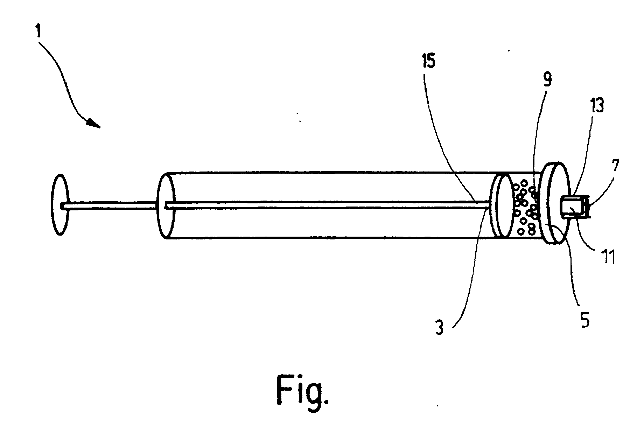

Die Figur zeigt:

Die Figur zeigt in schematischer Darstellung eine erfindungsgemäße Spritze.

### Beispiel 1:

Die Figur zeigt eine Spritze 1 aus Polystyrol mit einem Stempel 3, einem abschraubbaren Verschluß 5 mit einem Verschlußansatz 13 (male Luer) und einer auf dem Verschlußansatz 13 angeordneten und diesen abschließenden abnehmbaren Kappe 7 mit Septum. Der Stempel 3 weist eine Sollbruchstelle 15 auf. Dargestellt ist auch mit nicht dargestelltem IgG beschichtetes Granulat 9 aus Polystyrol. Die Größe der Granulatpartikel 9 liegt zwischen 1 und 3 mm Durchmesser, wobei jedoch auch kleinere Partikel, insbesondere größer als 100 µm, eingesetzt werden können.

Zur Herstellung der Spritze 1 wird das Granulat 9 mit einem handelsüblichen IgG-Präparat (Venimmun®, Behring) beschichtet, indem das IgG-Präparat in die Spritze aufgenommen und das Granulat sowie die Spritzeninnenwand damit benetzt wird. Anschließend wird die Spritze 1 bei Raumtemperatur mindestens 15 Minuten inkubiert, um eine vollständige Bindung des IgG-Präparats an das Granulat 9 und die Spritzeninnenwand zu gewährleisten. Schließlich wird Heparin (Liquemin N 2500, Heparin-Natrium 2500 I.E.) oder Citrat (ACDA) in die Spritze eingebracht, um eine Koagulation des später aufgenommenen Blutes zu verhindern.

Die Spritze 1 wird eingesetzt, indem Blut eines Patienten mit Hilfe eines nicht dargestellten Adapters entnommen wird, der die abschraubbare Kappe 7 mittels eines nicht dargestellten Schlauches mit einer nicht dargestellten Kanüle verbindet. Der Adapter weist eine Nadel auf, mittels derer das im Verschlußansatz 13 vorhandene Septum durchstochen wird. Anschließend wird der Adapter abgenommen und eine Inkubation des Vollblutes bei 37°C für 24 Stunden unter dem Schutz der abnehmbaren Kappe 7, deren Septum sich selbsttätig geschlossen hat, durchgeführt. Die Inkubation kann stehend oder liegend erfolgen. Erfolgt die Inkubation stehend, wird das Plasma durch das Septum und einen sterilen Vorsatzfilter (0,2 µm) abgenommen. Zusätzlich oder alternativ kann eine Zentrifugation vorgesehen werden. Erfolgt die Inkubation liegend, wird das Blut zentrifugiert und das Plasma durch einen sterilen Vorsatzfilter (0,2 µm) abgenommen. Es kann aber auch vorgesehen sein, das Plasma durch das Septum abzunehmen ohne eine Zentrifugation durchzuführen. Anschließend wird das Plasma zum Beispiel an einer Nervenwurzel oder einem Gelenk des Patienten reinjiziert.

### Beispiel 2: Spritze mit Granulat

Steriles Granulat aus Polystyrol, Glas oder einem anderen nicht-pyrogenen, proteinbindenden Material wird unter sterilen Bedingungen im Batch-Verfahren mit Protein (zum Beispiel eine nach AMG zugelassene IgG-Präparation (zum Beispiel Venimmun®, Behring) in sterilem Wasser oder wässrigem Puffer auf 10 bis 100 µm/ml verdünnt) beschichtet und anschließend getrocknet.

Eine herkömmliche Polypropylenspritze (5, 10, 20 oder 50 ml) wird mit dem proteinbeschichteten Granulat (1, 2, 4 oder 10 cm³) sowie mit einer hinreichenden Menge eines Antikoagulans wie Heparin (Liquemin, Heparin-Natrium 2500 I.E.) oder Citrat (zum Beispiel ACDA) befüllt.

Die befüllte Spritze wird inklusive Abnahmekanüle und Schlauch verpackt und anschließend Gamma- oder Elektronen-sterilisiert. Auf diese Weise wird Pyrogenfreiheit gewährleistet.

Der Anwender entnimmt das sterile Besteck und entnimmt dem Patienten Blut. Die Spritze verfügt an ihrer Öffnung in dem Verschlußansatz über ein Septum, welches zur Entnahme durch das Abnahmezubehör, also die Nadel des Adapters, durchstochen wird. Nach Abnahme des Adapters verschließt sich das Septum selbsttätig wieder. Nach Blutentnahme wird der Spritzenstempel an einer Sollbruchstelle abgebrochen.

Die Spritze mit Blut wird 24 Stunden bei 37°C bis 41°C inkubiert.
a) Erfolgt die Inkubation stehend, wird das Plasma durch das Septum und einen sterilen Vorsatzfilter, zum Beispiel 0,2 µm, abgenommen.
b) Erfolgt die Inkubation liegend, wird nach Zentrifugation der Spritze das Plasma durch einen sterilen Vorsatzfilter, zum Beispiel 0,2 µm, abgenommen.

Die Reinjektion des Plasma erfolgt zum Beispiel an einer Nervenwurzel, ins Gelenk oder in die Bandscheibe.

### Beispiel 3: Spritze ohne Granulat

Eine Spritze aus einem nicht pyrogenen, proteinbindenden Material (5, 10, 20 oder 50 ml) wird steril mit Protein (zum Beispiel eine kommerziell nach AMG zugelassene IgG-Präparation (Venimmun®, Behring), gegebenenfalls in sterilem Wasser oder wässrigem Puffer auf 10 bis 100 µm/ml verdünnt) beschichtet. Vorzugsweise besteht die Spritze aus Polystyrol, Glas oder einem speziell modifizierten anderen Material.

Die beschichtete Spritze wird mit einer hinreichenden Menge Heparin (Liquemin, Heparin-Natrium 2500 I.E.) oder Citrat (ACDA) befüllt.

Die beschichtete, befüllte Spritze wird inklusive Abnahmekanüle und Schlauch anschließend Gamma- oder Elektronen-sterilisiert. Auf diese Weise wird Pyrogenfreiheit gewährleistet.

Der Anwender entnimmt das sterile Besteck und entnimmt dem Patienten Blut. Die Spritze verfügt an der Öffnung über ein in dem Verschlußansatz enthaltendes Septum, welches zur Entnahme durch das Abnahmezubehör, also den eine Nadel aufweisende Adapter durchstochen wird. Nach Abnahme des Adapters verschließt sich das Septum selbsttätig wieder. Nach Blutentnahme wird der Spritzenstempel an einer Sollbruchstelle abgebrochen.

Die Spritze mit Blut wird 24 Stunden bei 37°C bis 41°C inkubiert.
a) Erfolgt die Inkubation stehend (zum Beispiel in einem Reagenzglasständer), wird das Plasma durch das Septum abgenommen, wobei eine Filtration durch einen sterilen Vorsatzfilter, zum Beispiel 0,2 µm, erfolgt.
b) Erfolgt die Inkubation liegend, wird nach Zentrifugation der Spritze das Plasma durch das Septum abgenommen, wobei dabei durch einen sterilen Vorsatzfilter, zum Beispiel 0,2 µm, eine Filtration erfolgt.

Die Reinjektion des Plasma erfolgt zum Beispiel an einer Nervenwurzel oder ins Gelenk oder in die Bandscheibe.

### Beispiel 4: Herstellung des Interleukin-1 Rezeptor-Antagonisten in einer Spritze unter Verwendung von Heparin

Eine kommerziell erhältliche und arzneimittelrechtlich zugelassene Immunglobulin G-Präparation (Venimmum®, Behring) wird in sterilem wässrigem Puffer auf 10 bis 100 µg/ml verdünnt.

Diese Lösung wird in eine sterile Spritze aus Polystyrol gefüllt, deren innere Oberfläche effizient Protein bindet. Anschließend wird bei Raumtemperatur eine Inkubation von mindestens 15 Minuten zur Absättigung der inneren Wandfläche mit dem Immunglobulin G durchgeführt. Die Inkubationszeit kann auch mehr als 24 Stunden betragen.

Nach Abschluß der Inkubation und damit nach Adhäsion des Immunglobulins G in der inneren Oberfläche der Spritze wird die Immunglobulin G-Lösung aus der Spritze entfernt und die Spritze steril zwischengelagert. Arzneimittelrechtlich zugelassenes Heparin (Liquemin, Heparin-Natrium 2500 I.E.) wird in die beschichtete Spritze als Anticoagulanz aufgezogen.

Anschließend wird dem Patienten venöses Blut mit der beschichteten Spritze steril entnommen.

Die Spritze wird bei Raumtemperatur 12 bis 72 Stunden inkubiert. In dieser Zeit findet eine starke Anreicherung im Plasma enthaltender Proteine, insbesondere des Interleukin-1 Rezeptor-Antagonisten, im Blutplasma statt. Es konnte eine Konzentration von 1 bis 50 ng/ml des Interleukin-1 Rezeptor-Antagonisten festgestellt werden.

Anschließend wird das Blut oder das Plasma dem Patienten mit der beschichteten Spritze injiziert.

### Beispiel 5: Herstellung des Interleukin-1 Rezeptor-Antagonisten in einer Spritze

Eine kommerziell erhältliche und arzneimittelrechtlich zugelassene Immunglobulin G-Präparation (Venimmun®, Behring) wird in sterilem wässrigem Puffer auf 10 bis 100 µg/ml verdünnt.

Diese Lösung wird in eine sterile Polystyrolspritze gefüllt, deren Wandmaterial effizient Proteine bindet. Eine Inkubation bei Raumtemperatur über mindestens 15 Minuten, die der Absättigung der Innenwandfläche mit dem Immunglobulin G dient, wird durchgeführt.

Nach Abschluß der Inkubation und Adhäsion des Immunglobulins an der Spritzeninnenwand wird die Immunglobulin G-Lösung abgenommen und die Spritze steril zwischengelagert.

Anschließend wird dem Patienten venöses Blut mit der Spritze steril entnommen.

Die Spritze wird bei Raumtemperatur über 12 bis 24 Stunden inkubiert. In dieser Zeit findet eine starke Anreicherung im Plasma enthaltender Proteine, insbesondere des Interleukin-1 Rezeptor-Antagonisten, im Blutplasma statt. Es konnte eine Konzentration von 1 bis 50 ng/ml des Interleukin-1 Rezeptor-Antagonisten gefunden werden.

Anschließend wird das verdünnte Blut oder der Kulturüberstand dem Patienten injiziert.

## Patentansprüche

1. Verfahren zur Herstellung mindestens eines therapeutisch wirksamen Proteins oder eines Proteingemisches in einer Spritze, wobei innere Strukturen der Spritze mit einem Induktor beschichtet werden, die Spritze mit einer Körperflüssigkeit gefüllt, inkubiert und das therapeutisch wirksame Protein in der Körperflüssigkeit gebildet wird.

2. Verfahren, nach Anspruch 1, wobei der Induktor ein Immunglobulin ist.

3. Verfahren nach einem der vorhergehenden Ansprüche, wobei das Immunglobulin Immunglobulin G (IgG) ist.

4. Verfahren nach einem der vorhergehenden Ansprüche, wobei das therapeutisch wirksame Protein der Interleukin-1 Rezeptor-Antagonist (IRAP), Interleukin 4, Interleukin 10 oder löslicher Tumor-Nekrose-Faktor-Rezeptor Typ I oder Typ II ist.

5. Verfahren nach einem der vorhergehenden Ansprüche, wobei die inneren Strukturen der Spritze durch ihre innere Oberfläche gebildet werden.

6. Verfahren nach einem der vorhergehenden Ansprüche, wobei die inneren Strukturen auch durch in der Spritze vorhandene Kugeln, Gele, Glaswolle, Granulate oder Partikel gebildet werden.

7. Verfahren nach einem der vorhergehenden Ansprüche, wobei die inneren Strukturen aus Polystyrol oder Glas bestehen oder dieses enthalten.

8. Verfahren nach einem der vorhergehenden Ansprüche, wobei die Spritze vor dem Inkubieren zusätzlich mit Anticoagulantien, vorzugsweise Heparin, versehen wird.

9. Verfahren nach einem der vorhergehenden Ansprüche, wobei die Körperflüssigkeit Blut ist.

10. Verfahren zur Herstellung einer zur In-vitro-Induktion von therapeutisch wirksamen Proteinen geeigneten Spritze, wobei Induktoren, insbesondere Immunglobuline, in eine Spritze mit proteinbindenden inneren Strukturen gegeben und so inkubiert werden, daß die Induktoren, insbesondere Immunglobuline, an die inneren Strukturen binden.

11. Verfahren nach Anspruch 10, wobei die inneren Strukturen der Spritze aus Polystyrol oder Glas bestehen oder dieses enthalten.

12. Verfahren nach einem der Ansprüche 10 oder 11, wobei das Immunglobulin IgG ist.

13. Verfahren nach einem der Ansprüche 10 bis 12, wobei das therapeutisch wirksame Protein IRAP ist.

14. Spritze mit inneren Strukturen, insbesondere hergestellt nach einem der Ansprüche 10 bis 13, **dadurch gekennzeichnet, daß** die inneren Strukturen der Spritze aus Polystyrol oder Glas bestehen oder dieses enthalten und mit einem Induktor, insbesondere Immunglobulin G, beschichtet sind.

## Claims

1. Process for preparing at least one therapeutically active protein or a protein mixture in a syringe, where internal structures in the syringe are coated with an inducer, the syringe is filled with a body fluid and incubated, and the therapeutically active protein is formed in the body fluid.

2. Process according to Claim 1, where the inducer is an immunoglobulin.

3. Process according to either of the preceding claims, where the immunoglobulin is immunoglobulin G (IgG).

4. Process according to one of the preceding claims, where the therapeutically active protein is interleukin 1 receptor antagonist (IRAP), interleukin 4, interleukin 10 or soluble tumour necrosis factor receptor type I or type II.

5. Process according to one of the preceding claims, where the internal structures in the syringe are formed by its internal surface.

6. Process according to one of the preceding claims, where the internal structures are also formed by spheres, gels, glass wool, granules or particles which are present in the syringe.

7. Process according to one of the preceding claims, where the internal structures consist of polystyrene or glass or contain polystyrene or glass.

8. Process according to one of the preceding claims, where, before being incubated, the syringe is additionally provided with anticoagulants, preferably heparin.

9. Process according to one of the preceding claims, where the body fluid is blood.

10. Process for producing a syringe which is suitable for the in-vitro induction of therapeutically active proteins, where inducers, in particular immunoglobulins, are introduced into a syringe possessing protein-binding internal structures and incubated such that the inducers, in particular immunoglobulins, bind to the internal structures.

11. Process according to Claim 10, where the internal structures in the syringe consist of polystyrene or glass or contain polystyrene or glass.

12. Process according to either Claim 10 or 11, where the immunoglobulin is IgG.

13. Process according to one of Claims 10 to 12, where the therapeutically active protein is IRAP.

14. Syringe which possesses internal structures and which is produced, in particular, according to one of Claims 10 to 13, **characterized in that** the internal structures in the syringe consist of polystyrene or glass or contain polystyrene or glass and are coated with an inducer, in particular immunogloblin G.

## Revendications

1. Procédé de production d'au moins une protéine ou d'un mélange de protéines thérapeutiquement active(s) dans une seringue, selon lequel des structures internes de la seringue sont revêtues avec un inducteur, la seringue est remplie d'un fluide corporel, et mise en incubation et la protéine thérapeutiquement active est formée dans le fluide corporel.

2. Procédé selon la revendication 1,
selon lequel
l'inducteur est une immunoglobuline.

3. Procédé selon l'une des revendications précédentes,
selon lequel
l'immunoglobuline est l'immunoglobuline G(IgG).

4. Procédé selon l'une des revendications précédentes,
selon lequel
la protéine active thérapeutiquement est l'interleukine-1, l'antagoniste de récepteur (IRAP), l'interleukine-4, l'interleukine-10, ou le récepteur soluble de facteur de nécrose des tumeurs de type I ou de type II.

5. Procédé selon l'une des revendications précédentes,
selon lequel
les structures internes de la seringue sont formées par leurs surfaces internes.

6. Procédé selon l'une des revendications précédentes,
selon lequel
les structures internes sont formées également par des billes, des gels, de la laine de verre, des granulés ou des particules présentes dans la seringue.

7. Procédé selon l'une des revendications précédentes,
selon lequel
les structures internes consistent en du polystyrène ou du verre ou contiennent celui-ci.

8. Procédé selon l'une des revendications précédentes,
selon lequel la seringue est équipée avant la mise en incubation en supplément avec des anticoagulants, de préférence de l'héparine.

9. Procédé selon l'une des revendications précédentes,
selon lequel
le fluide corporel est le sang.

10. Procédé de production d'une seringue appropriée pour l'induction « in vitro » de protéines thérapeutiquement actives,
selon lequel
des inducteurs - en particulier des immunoglobulines - sont administrés dans une seringue ayant des structures intérieures reliant des protéines et ainsi sont mises en incubation de façon que les inducteurs - en particulier des immunoglobulines - se lient aux structures internes.

11. Procédé selon la revendication 10,
selon lequel
les structures internes de la seringue consistent en du polystyrène ou du verre ou renferment celui-ci.

12. Procédé selon l'une des revendications 10 ou 11,
selon lequel
l'immunoglobuline est un IgG.

13. Procédé selon l'une des revendications 10 à 12,
selon lequel la protéine thérapeutiquement active est l'IRAP.

14. Seringues ayant des structures internes, en particulier produites selon l'une quelconque des revendications 10 à 13,
**caractérisées en ce que**
les structures internes des seringues sont du polystyrène ou du verre ou renferment celui-ci, et sont revêtues d'un inducteur en particulier de l'immunoglobuline G.
